# EUROPÄISCHE PATENTSCHRIFT

(11) **EP 0 834 328 B1**
(45) Veröffentlichungstag und Bekanntmachung des Hinweises auf die Patenterteilung: **12.11.2003**
(21) Anmeldenummer: 97113346.7
(22) Anmeldetag: 01.08.1997
(51) Int. Cl.: A61M 1/16

(54) **Dialysegerät mit Mitteln zur Entkalkung des Dialysierflüssigkeitskreislaufs sowie Verfahren zur Bestimmung seines Verkalkungsgrades**
Dialysis machine with means for decalcification of the dialysate circuit and method for determining its degree of calcification
Appareil de dialyse avec moyens de détartrage du cricuit de dialysat et méthode pour déterminer son niveau de calcification

(30) Priorität: 06.09.1996 DE 19636255
(43) Veröffentlichungstag der Anmeldung: 08.04.1998
(60) Teilanmeldung: 02022117.2
(73) Patentinhaber: Fresenius Medical Care Deutschland GmbH, 61352 Bad Homburg (DE)
(72) Erfinder: Spickermann, Reiner, 97535 Wasserlosen/Burghausen (DE)
(74) Vertreter: Laufhütte, Dieter, Dr.-Ing.

(56) Entgegenhaltungen:
- EP-A- 0 648 508
- US-A- 4 153 554
- US-A- 5 326 476

## Beschreibung

Die vorliegende Erfindung betrifft ein Dialysegerät zur Entfernung von toxischen Substanzen aus Blut mit Mitteln zur Entkalkung eines Dialysierflüssigkeitskreislaufs. Die Erfindung betrifft ferner Verfahren zur Bestimmung des Verkalkungsgrades eines Dialysegeräts.

Bei der Bicarbonatdialyse zur Entfernung von toxischen Substanzen aus Blut tritt das Problem auf, daß Bicarbonat mit Calcium einen schwerlöslichen Niederschlag bildet. Die Komponenten werden daher bei der Bicarbonatdialyse als Natriumbicarbonat in der basischen Komponente, und als Calciumchlorid, Bestandteil der sauren Komponente, getrennt gehalten, und erst in der Maschine selbst oder kurz vor Anwendung gemischt.

Trotz dieser Maßnahmen bilden sich in der Dialysemaschine Kristallisationskeime, was mit der Zeit zu einer stärkeren Ablagerung von Calciumcarbonat führt. Aus diesem Grunde ist es notwendig, eine Dialysemaschine von Zeit zu Zeit zu entkalken. Übliche Entkalkungsmittel sind beispielsweise Essigsäure oder Zitronensäure.

Aus der US 4 153 554 ist ein Dialysegerät bekannt, bei dem der Verschmutzungsgrad des Meßfensters eines Blutleckdetektors automatisch erfaßt wird. Nach Überschreiten eines entsprechenden Grenzwertes für den Verschmutzungsgrad wird das Meßfenster entweder während der Sterilisation des Dialysegerätes chemisch oder nach Entnahme des Meßfensters mechanisch gereinigt.

Aus der EP 0648 508 A1 ist es bekannt, einen manuell bedienbaren bzw. zeitgesteuerten Entkalkungszyklus einzuleiten.

Diese Entkalkungsverfahren erweisen sich insofern als nachteilig, als eine manuelle Auswahl eines Entkalkungsprogramms durch das Bedienpersonal vorgenommen werden muß, was sich in der Regel als sehr zeitaufwendig gestaltet. Ferner besteht bei der zeitgesteuerten Entkalkung der Nachteil, daß eine Entkalkung auch dann vorgenommen wird, wenn der Verkalkungsgrad eine derartige Entkalkung noch gar nicht erforderlich machen würde. Dies führt zu einer unnötigen Beanspruchung der Entkalkungsvorrichtung. Außerdem steht das Dialysegerät zur eigentlichen Behandlung während dieses Zeitraumes nicht zur Verfügung.

Aufgabe der Erfindung ist daher die Schaffung eines Dialysegeräts mit einer Entkalkungsvorrichtung, welche die obigen Nachteile vermeidet.

Die Erfindung hat ferner zum Ziel, Verfahren anzugeben, die in vorteilhafter Weise zur Bestimmung des Verkalkungsgrades eines Dialysegeräts verwendet werden können.

Erfindungsgemäß wird diese Aufgabe gelöst durch ein Dialysegerät mit den Merkmalen des Patentanspruchs 1.

Mit dem erfindungsgemäßen Dialysegerät wird eine Entkalkung lediglich in Abhängigkeit vom tatsächlich gemessenen Verkalkungszustand automatisch initiiert. Eine Beanspruchung des Bedienpersonals, sowie eine unnötige Betätigung der Entkalkungsmittel kann somit vermieden werden.

Bevorzugte Ausgestaltungen sind Gegenstand der Unteransprüche.

Gemäß einer vorteilhaften Weiterbildung ist der Verkalkungsgrad des Dialysegeräts über Mittel zur Bestimmung der Trübung eines Fensters eines Blutleckdetektors bestimmbar. Hierbei wird die Tatsache ausgenutzt, daß mit zunehmender Verkalkung eines Dialysierflüssigkeitskreislaufes sich auch am Fenster eines Blutleckdetektors des Dialysegeräts Kalkablagerungen bilden, die zu einer Abschwächung eines durch ein derartiges Fenster durchtretenden Lichtstrahls führen.

Gemäß einer weiteren vorteilhaften Ausbildung ist der Verkalkungsgrad des Dialysegeräts über Mittel zur Bestimmung der Trübung der verwendeten Dialysierflüssigkeit bestimmbar, welche eine zunehmende Verkalkung des Dialysatkreislaufs bzw. eine zunehmende Kalkablagerung ebenfalls bedingt. Dies stellt eine einfache und zuverlässige Methode zur Bestimmung des Verkalkungsgrades eines Dialysiergerätes dar, welche beispielsweise kontinuierlich, oder aber auch diskontinuierlich, d.h. in bestimmten Zeitintervallen, durchgeführt werden kann.

Besonders vorteilhaft ist es, einen Blutleckdetektor eines Dialysegeräts zu verwenden, der einen Signalausgang aufweist, der auf Trübungen der Dialysierflüssigkeit und/oder des Fensters des Blutleckdetektors reagiert. Dies stellt ein besonders einfaches Verfahren dar, bei dem lediglich ein vorhandener Blutleckdetektor mit einem weiteren Signalausgang ausgestattet werden muß.

Gemäß einer vorteilhaften Weiterbildung weist das Dialysegerät Mittel auf, die bei Feststellung eines vorbestimmten Verkalkungsgrades diese Information speichern und die automatische Entkalkung erst nach Beendigung einer Dialyse einleiten. Hierdurch ist gewährleistet, daß der Dialysebetrieb in keiner Weise behindert wird.

Gemäß einer weiteren Ausgestaltung der Erfindung ist vorgesehen, die für eine Bicarbonatdialyse verwendete saure Komponente als Entkalkungsmittel zu verwenden. Hiermit ist eine besonders einfache Möglichkeit zur Entkalkung eine Dialysegeräts zur Verfügung gestellt.

Gemäß einem erfindungsgemäßen Verfahren zur Bestimmung des Verkalkungsgrades eines Dialysegeräts wird die Trübung eines Fensters bzw. Sichtfensters eines Blutleckdetektors des Dialysegeräts bestimmt, und der derart ermittelte Wert in Beziehung gesetzt zu einem Verkalkungsgrad der Dialysemaschine. Dies erweist sich als vorteilhaft, da der Verkalkungsgrad und somit die Trübung eines Sichtfensters eines Blutleckdetektors abhängig vom Verkalkungsgrad des Dialysierflüssigkeitskreislaufs insgesamt.

Gemäß einem erfindungsgemäßen Verfahren zur Bestimmung des Verkalkungsgrades eines Dialysegeräts wird der Trübungsgrad einer verwendeten Dialysierflüssigkeit bestimmt, und der derart ermittelte Wert in Beziehung gesetzt zu einem Verkalkungsgrad des Dialysegeräts. Hierbei wird ausgenutzt, daß eine bestimmte Beziehung zwischen dem Trübungsgrad der verwendeten Dialysierflüssigkeit und dem aktuellen Verkalkungszustand eines Dialysegeräts besteht.

Besonders vorteilhaft hierbei ist, den Trübungsgrad mittels eines auf Trübungen reagierenden Signalausgangs eines Blutleckdetektors zu ermitteln. Hierzu ist es lediglich notwendig, einen herkömmlichen Blutleckdetektor mit einem Signalausgang bzw. einem Signalkanal zu versehen, der auf weitgehend farb-unspezifizierte Trübungen, beispielsweise verursacht durch eine Trübung eines Fensters und/oder der Dialysierflüssigkeit, reagiert. Bei Anordnung eines entsprechenden Sensors des Blutleckdetektors innerhalb des Dialysierflüssigkeitskreislaufs wird diese Trübung durch CaCO₃-Ablagerungen am Sensor selbst verursacht. Es ist jedoch gleichfalls möglich, einen derartigen Sensor an anderer Stelle des Dialysierflüssigkeitskreislaufs, anzuordnen.

Bevorzugte Ausführungsformen der Erfindung werden nun unter Bezug auf die beigefügte Zeichnung näher erläutert. Es zeigt:

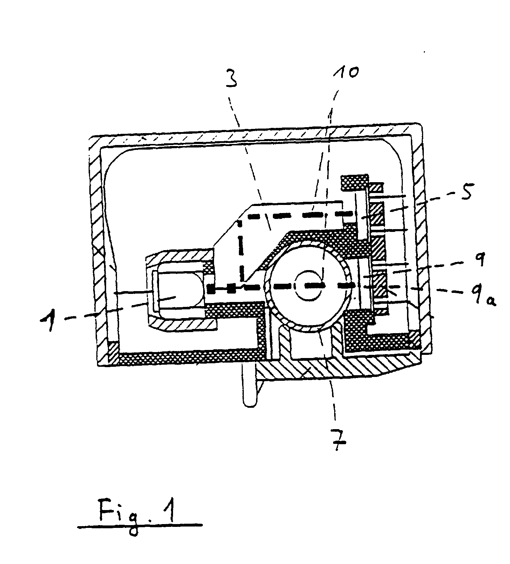

- Fig. 1:: schematisch den Aufbau eines Blutleckdetektors, welcher gemäß einer bevorzugten Ausführungsform der Erfindung zur Feststellung eines Ver kalkungsgrades eines Dialysegerätes verwendbar ist.

Ein Blutleckdetektor weist beispielsweise eine Bicolor-LED 1 auf, welcher alternierend orange-rotes und grünes Licht (Lichtstrahl 1) abstrahlt. Ca. 50% dieses Lichtes wird über ein Prisma 3 ausgekoppelt und auf den Referenzempfänger 5 geleitet. Dieser Empfänger 5 bildet den Eingang eines Referenzkanals. Der Referenzkanal regelt die Lichtintensität der beiden Farben so, daß sie in einem festen Verhältnis zueinander stehen. Das restliche Licht durchstrahlt eine Küvette 7 und das darin befindliche Medium (z.B. Dialysierflüssigkeit). Anschließend trifft dieses Licht auf einen Meß- oder Küvettenempfänger 9. Befindet sich Blut in der Dialysierflüssigkeit, erfährt das grüne Licht beim Durchstrahlen der Küvette einen Intensitätsverlust. Das dadurch geänderte Intensitätsverhältnis wird in einem sich an den Meßempfänger 9 anschließenden Meßkanal ausgewertet.

Gemäß einer bevorzugten Ausführungsform der vorliegenden Erfindung weist ein Dialysegerät einen Blutleckdetektor auf, der mit einem zusätzlichen Signalausgang bzw. Signalkanal ausgerüstet ist, der auf Trübungen reagiert (Trübungsempfänger 9a bzw. Trübungskanal). Eine elektronische Schaltung bzw. deren Abgleich ist hierbei bevorzugt so ausgelegt, daß bei Nicht-Vorliegen einer Trübung eine Ausgangsspannung von 5 Volt anliegt. Bei zunehmender Verkalkung während einer Bicarbonatdialyse nimmt diese Ausgangsspannung aufgrund zunehmender Trübung ab. Unterschreitet diese Spannung einen bestimmten Schwellwert, z.B. 3,5 Volt, wird automatisch ein Entkalkungszyklus initiiert. Hierzu kann beispielsweise ein mikroprozessorgesteuertes Ventil derart betätigt werden, daß ein geeignetes Entkalkungsmittel in den Dialysierflüssigkeitskreislauf eingeführt wird. Insbesondere ist es hierbei möglich, beispielsweise durch Rechnersteuerung des Systems, ein Flag bzw. ein Triggersignal für einen Entkalkungszyklus zu setzen bzw. zu erzeugen, wobei dann die tatsächliche Entkalkung beispielsweise erst nach der Dialyse eingeleitet wird.

Das Vorliegen von Blut in der Dialysierflüssigkeit wird also über einen Meßkanal, und das Auftreten einer Trübung der Dialysierflüssigkeit über einen Trübungskanal festgestellt. Die Signalverarbeitung des Meßkanals bzw. Blutleckkanals erfolgt dadurch, daß eine Fotozelle die von der Zweifarben-LED empfangenen Lichtsignale in eine bezüglich der empfangenen Lichtstärke logarithmische Spannung umwandelt, so daß die aus der alternierenden Rot-Grün-Beleuchtung erzeugte Rechteckamplitude dem Logarithmus des Quotienten rot/grün proportional ist und farbunspezifische Trübungen, die beide Farben gleichermaßen dämpfen, das Signal nicht beeinflussen. Befindet sich jedoch Blut in der Dialysierflüssigkeit, erfährt, wie oben beschrieben das grüne Licht einen Intensitätsverlust, so daß das geänderte Verhältnis vom Meßkanal ausgewertet werden kann. Der Trübungskanal ist derart ausgelegt, daß seine Ausgangsspannung bei zunehmender Trübung immer weiter abnimmt.

Gemäß einer weiteren bevorzugten Ausführungsform wird der Verkalkungsgrad eines Dialysegeräts durch Auswertung bestimmter Parameter einer zur Förderung der Dialysierflüssigkeit verwendeten Zahnradpumpe bestimmt.

Die Ausführungsform weist Mittel auf, über welche bei Feststellung eines vorbestimmten Verkalkungsgrades des Dialysiergeräts eine automatische Entkalkung eingeleitet wird. Hierbei sind beispielsweise Entkalkungsmittel wie Essigsäure, Peressigsäure oder Zitronensäure verwendbar.

Gemäß einer bevorzugten Ausführungsform wird die bei der Bicarbonatdialyse verwendete saure Komponente auch zur Entkalkung verwendet. Dies ist möglich, da in dieser Komponente meist Acetat enthalten ist. Hiermit können komplette Desinfektionszyklen mit anderen kalklösenden Desinfektionsmitteln weitgehend vermieden werden.

Wesentliche Vorteile sind hier, daß weder ein zusätzliches Reservoir für das Entkalkungsmittel noch eine zusätzliche Vorrichtung zur Förderung des Entkalkungsmittels benötigt wird. Ferner ist vor Beginn des Entkalkungszyklus keine Konnektion bzw. Änderung einer Konnektion am Dialysegerät vorzunehmen, da die saure Komponente bereits am Dialysegerät angeschlossen ist, sei es über Versorgung mittels Kanister oder über eine zentrale Versorgungseinrichtung. Das Dialysegerät kann dann bei Vorliegen der o.g. Kriterien für eine Entkalkung beispielsweise nach Erkennen des Zustandes "Dialyseende" vollautomatisch einen Entkalkungszyklus einleiten.

Erfindungsgemäß ist daher eine Entkalkung eines Dialysegeräts derart vereinfacht, daß eine einfache, bedienerfreundlich und sichere Regenerierung der Dialysemaschine gewährleistet ist. Hierbei ist sowohl der Erfolg der Entkalkung selbst, als auch die Patientensicherheit gewährleistet.

Zur weiteren Erhöhung der Sicherheit ist es möglich, Mittel vorzusehen, die nach Beendigung eines Entkalkungszyklus in der Lage sind, Reste des verwendeten Entkalkungsmittels zu detektieren, so daß das Verbleiben von Entkalkungsmittelresten in dem Dialysegerät wirksam vermieden werden kann.

## Patentansprüche

1. Dialysegerät zur Entfernung von toxischen Substanzen aus biologischen Flüssigkeiten mit Mitteln zur Entkalkung eines Dialysierflüssigkeitskreislaufs und Mitteln (1) zur Messung des Verkalkungsgrades des Dialysegeräts
**dadurch gekennzeichnet,**
**daß** die Mittel (1) zur Messung des Verkalkungsgrades bei Feststellung eines vorbestimmten Verkalkungsgrades des Dialysegeräts eine automatische Entkalkung des Dialysierflüssigkeitskreislaufs des Dialysegeräts einleiten.

2. Dialysiergerät nach Anspruch 1, **dadurch gekennzeichnet, daß** der Verkalkungsgrad des Dialysegeräts über Mittel (1) zur Bestimmung der Trübung eines Fensters eines Blutleckdetektors bestimmbar ist.

3. Dialysegerät nach Anspruch 1, **dadurch gekennzeichnet, daß** der Verkalkungsgrad des Dialysegeräts über Mittel (1) zur Bestimmung der Trübung der verwendeten Dialysierflüssigkeit bestimmbar ist.

4. Dialysegerät nach Anspruch 2 oder 3, **dadurch gekennzeichnet, daß** es einen Blutleckdetektor mit einem Signalausgang aufweist, der auf Trübungen der Dialysierflüssigkeit und/oder eines Fensters eines Blutleckdetektors reagiert.

5. Dialysegerät nach einem der vorstehenden Ansprüche, **dadurch gekennzeichnet, daß** Mittel vorgesehen sind, die bei Feststellung des vorbestimmten Verkalkungsgrades diese Information speichern, und die automatische Entkalkung erst nach Beendigung einer Patientendialyse einleiten.

6. Dialysegerät nach einem der vorstehenden Ansprüche, **dadurch gekennzeichnet, daß** die bei einer Bicarbonatdialyse verwendete saure Komponente als Entkalkungsmittel verwendbar ist.

7. Verfahren zur Bestimmung des Verkalkungsgrades eines Dialysegerätes nach einem der Ansprüche 1-6, wobei die Trübung eines Sichtfensters eines Blutleckdetektors bestimmt wird, und der derart ermittelte Wert in Beziehung gesetzt wird zu einem Verkalkungsgrad des Dialysegeräts.

8. Verfahren zur Bestimmung des Verkalkungsgrades eines Dialysegeräts nach einem der Ansprüche 1-6, wobei der Trübungsgrad einer verwendeten Dialysierflüssigkeit bestimmt wird, und der derart ermittelte Wert in Beziehung gesetzt wird zu einem Verkalkungsgrad des Dialysegeräts.

9. Verfahren nach einem der Ansprüche 7 oder 8, wobei der Trübungsgrad des Dialysegeräts mittels eines auf Trübungen reagierenden Signalausgangs eines Blutleckdetektors ermittelt wird.

## Claims

1. Dialysis machine for removing toxic substances from biological fluids, with means for decalcification of a dialysate circuit and means (1) for measuring the degree of calcification of the dialysis machine, **characterized in that** the means (1) for measuring the degree of calcification initiate automatic decalcification of the dialysate circuit of the dialysis machine when a predetermined degree of calcification of the dialysis machine is detected.

2. Dialysis machine according to Claim 1, **characterized in that** the degree of calcification of the dialysis machine can be determined via means (1) for determining the clouding of a window of a blood leak detector.

3. Dialysis machine according to Claim 1, **characterized in that** the degree of calcification of the dialysis machine can be determined, via means (1) for determining the clouding of the dialysate used.

4. Dialysis machine according to Claim 2 or 3, **characterized in that** it has a blood leak detector with a signal output which reacts to clouding of the dialysate and/or of a window of a blood leak detector.

5. Dialysis machine according to one of the preceding claims, **characterized in that** means are provided which, when the predetermined degree of calcification is detected, store this information and initiate automatic decalcification only after a patient dialysis session has ended.

6. Dialysis machine according to one of the preceding claims, **characterized in that** the acid component used in bicarbonate dialysis can be used as decalcification agent.

7. Method for determining the degree of calcification of a dialysis machine according to one of Claims 1-6, where the clouding of a viewing window of a blood leak detector is determined, and the value thus obtained is correlated with a degree of calcification of the dialysis machine.

8. Method for determining the degree of calcification of a dialysis machine according to one of Claims 1-6, where the degree of clouding of a dialysate is determined, and the value thus obtained is correlated with a degree of calcification of the dialysis machine.

9. Method according to either of Claims 7 and 8, where the degree of clouding of the dialysis machine is determined by means of a signal output of a blood leak detector reacting to clouding.

## Revendications

1. Appareil de dialyse pour retirer des substances toxiques de liquides biologiques avec des moyens de détartrage d'un circuit de dialysat et de moyens (1) pour mesurer le degré de calcification de l'appareil de dialyse, **caractérisé en ce que** les moyens (1) pour mesurer le degré de calcification, lors de la constatation d'un degré de calcification prédéterminé de l'appareil de dialyse, déclenchent un détartrage automatique du circuit de dialysat de l'appareil de dialyse.

2. Appareil de dialyse selon la revendication 1, **caractérisé en ce que** le degré de calcification de l'appareil de dialyse peut être déterminé par des moyens (1) pour déterminer le trouble d'une fenêtre d'un détecteur de fuite de sang.

3. Appareil de dialyse selon la revendication 1, **caractérisé en ce que** le degré de calcification de l'appareil de dialyse peut être déterminé par des moyens (1) pour déterminer le trouble du dialysat utilisé.

4. Appareil de dialyse selon la revendication 2 ou 3, **caractérisé en ce qu'**il comporte un détecteur de fuite de sang avec une sortie de signaux qui réagit à des troubles du dialysat et/ou d'une fenêtre du détecteur de fuite de sang.

5. Appareil de dialyse selon l'une des revendications précédentes, **caractérisé en ce que** des moyens sont prévus qui, lors de la constatation d'un degré de calcification prédéterminé stockent ces informations et déclenchent le détartrage automatique seulement à la fin d'une dialyse d'un patient.

6. Appareil de dialyse selon l'une des revendications précédentes, **caractérisé en ce que** la composante acide utilisée lors d'une dialyse au bicarbonate est utilisable comme moyen de détartrage.

7. Procédé pour déterminer le degré de calcification d'un appareil de dialyse selon l'une des revendications 1 à 6, où le trouble d'une fenêtre de vision d'un détecteur de fuite de sang est déterminé, et la valeur ainsi obtenue est mise en relation avec un degré de calcification de l'appareil de dialyse.

8. Procédé pour déterminer le degré de calcification d'un appareil de dialyse selon l'une des revendications 1 à 6, où le degré de trouble d'un dialysat utilisé est déterminé, et la valeur ainsi obtenue est mise en relation avec un degré de calcification de l'appareil de dialyse.

9. Procédé selon l'une des revendications 7 ou 8, où le degré du trouble de l'appareil de dialyse est déterminé au moyen d'une sortie de signaux d'un détecteur de fuite de sang réagissant à des troubles.
